# EUROPÄISCHE PATENTSCHRIFT

(11) **EP 2 684 498 B1**
(45) Veröffentlichungstag und Bekanntmachung des Hinweises auf die Patenterteilung: **08.11.2017**
(21) Anmeldenummer: 13003367.3
(22) Anmeldetag: 03.07.2013
(51) Int. Cl.: A47K 10/48

(54) **Händetrockner**
Hand dryer
Sèche-mains

(30) Priorität: 11.07.2012 DE 202012006801 U
(43) Veröffentlichungstag der Anmeldung: 15.01.2014
(73) Patentinhaber: ELECTROSTAR GmbH, 73262 Reichenbach/Fils (DE)
(72) Erfinder: Gorovoy, Alexey, Krasnogorsky Rayon, Moscow 143405 (RU)
(74) Vertreter: Kohl, Karl-Heinz

(56) Entgegenhaltungen:
- WO-A1-80/01983
- WO-A1-2011/074018
- CH-A- 379 717
- CN-U- 202 288 092
- DE-A1- 19 905 985
- JP-A- H09 135 790
- JP-A- 2000 316 747
- JP-A- 2005 052 352
- US-A1- 2011 171 083

## Beschreibung

Die Erfindung betrifft einen Händetrockner nach dem Oberbegriff des Anspruches 1.

Im Krankenhausbereich ist es üblich, dass sich der Arzt die Hände mit Seife wäscht und sie anschließend mit dem Handtuch oder mit dem Händetrockner trocknet. Daran anschließend wird eine Desinfektionslösung aus einem Armspender auf die Hände gespült. Der Arzt muss so lange warten, bis die Hände trocken sind, bevor er sterile Handschuhe anziehen kann. Dieser Ablauf ist sehr zeitaufwendig und benötigt in der Regel drei bis vier Minuten. Bei der Arbeit in Manipulationsräumen muss der Arzt zwischendurch erneut Desinfektionslösung auf die Hände spülen und dann warten, bis sie trocken sind.

Erschwerend kommt hinzu, dass sich das Waschbecken, der Händetrockner bzw. das Handtuch und der Desinfektionsmittelspender häufig auf unterschiedlichen Höhen und an verschiedenen Stellen befinden. Dies trägt zur zeitaufwendigen Desinfektion bei.

Es ist ein Händetrockner bekannt (JP H09 135790 A), bei dem die Austrag-öffnungen für das Desinfektionsmittel in einem Bereich vorgesehen sind, in dem der Strömungsraum konstanten Querschnitt hat. Die Mitnahme des Desinfektionsmittels durch die durch den Strömungsraum strömende Luft ist darum ungenügend.

Es ist weiter bekannt (WO 2011/074018 A1), einen Wasserhahn mit einer zusätzlichen Leitung zum Austrag von Seife oder einem Desinfektionsmittel sowie mit einem Auslass für Trocknungsluft zu versehen. Der Hahn befindet sich im Bereich oberhalb eines Waschbeckens.

Ein weiterer bekannter Händetrockner (JP 2005/052352 A) hat einen Tank für ein Desinfektionsmittel, das über eine Düse ausgetragen wird. Sie befindet sich in einer Wand eines Gehäuses, in dem ein Gebläse zur Erzeugung des Trocknungsluftstromes untergebracht ist.

Aus der CH 379 717 ist ein Lufttrockengerät bekannt, das mit einem Gehäuse versehen ist, in dem sich ein Elektromotor befindet, auf dessen Motorwelle zwei Flügellüfter sitzen. Mit ihnen wird die aus dem Gehäuse austretende Trocknungsluft erzeugt, die über jeweils eine Heizeinrichtung nach außen geblasen wird. Das Lufttrockengerät kann mit einem Ultraviolettstrahler oder einem desinfizierenden Duftspender zur Desinfektion der Trockenluft sowie der zu trocknenden Hände versehen sein.

Ein anderer bekannter Händetrockner (DE 199 05 985 A1) hat ein Gehäuse, in dem zwei winklig zueinander liegende Verteilerplatten untergebracht sind. Sie begrenzen zusammen mit benachbarten Gehäusewänden Strömungskanäle für die Trocknungsluft.

Bei einem weiteren bekannten Händetrockner (JP 2000/316747 A) werden die Trocknungsluft und das Desinfektionsmittel räumlich getrennt voneinander unmittelbar auf die zu trocknenden Hände geleitet.

Bei einem weiteren bekannten Händetrockner (WO 80/01983 A1) wird die Trocknungsluft über eine Wandung des Gehäuses in einen Nassraum eingebracht, in den die zu trocknenden Hände gesteckt werden. An die Wände des Nassraumes kann ein Desinfektionsmittel gesprüht werden. Dieser Händetrockner umfasst alle Merkmale des Oberbegriffs des Anspruchs 1.

Es ist schließlich ein Händetrockner bekannt (US 2011/0171083 A1), bei dem über eine Leitung Druckluft von einem Kompressor zum Luftauslass gefördert wird. In den Luftstrom wird ein Desinfektionsmittel eingebracht.

Der Erfindung liegt die Aufgabe zugrunde, den gattungsgemäßen Händetrockner so auszubilden, dass eine Händedesinfektion innerhalb kurzer Zeit zuverlässig möglich ist.

Diese Aufgabe wird beim gattungsgemäßen Händetrockner erfindungsgemäß mit den kennzeichnenden Merkmalen des Anspruches 1 gelöst.

Beim erfindungsgemäßen Händetrockner wird das Desinfektionsmittel dem Luftstrom zugeführt, mit dem die Trocknung der Hände durchgeführt wird. Das Desinfektionsmittel wird über die wenigstens eine Austragöffnung ausgetragen. Der Anwender muss darum nicht unterschiedliche Stellen aufsuchen, um die Hände zu trocknen, zu desinfizieren und anschließend wieder zu trocknen. Durch den Einsatz des Händetrockners wird die Zeit zum Verdunsten der Desinfektionslösung sehr gering gehalten, so dass der gesamte Desinfektionsablauf nur wenig Zeit benötigt. Das Desinfektionsmittel kann gut verteilt auf die Hände aufgebracht werden. Um das Desinfektionsmittel optimal zu verteilen, ist die Austragöffnung an einer Düse vorgesehen, die an einer Wand einer Luftführung des Händetrockners vorhanden ist. Die Düse kann so ausgebildet werden, dass das Desinfektionsmittel sehr fein verteilt in den Luftstrom gelangt. In der Luftführung ist die Verteilerplatte vorgesehen, die im Strömungsweg der Luft liegt. Die Verteilerplatte begrenzt in vorteilhafter Weise zusammen mit der Wandung der Luftführung wenigstens einen Strömungsraum, dessen Querschnitt in Strömungsrichtung der Luft abnimmt. Dadurch kann in konstruktiv einfacher Weise eine hohe Beschleunigung des Luftstromes innerhalb der Luftführung erreicht werden. Die Düse für den Austrag des Desinfektionsmittels ist im Bereich dieses Strömungsraums angeordnet, so dass die aus der Düse austretenden Partikel von Desinfektionsmittel durch die beschleunigte Luft zuverlässig mitgenommen und im Luftstrom verteilt werden. Dabei ist die Düse in einem Bereich der Luftführung vorgesehen, in dem die Luft beschleunigt wird. Durch die hohe Luftgeschwindigkeit erfolgt eine optimale Vermischung der aus der Düse austretenden Teilchen des Desinfektionsmittels mit der Luft. Dies trägt zu einer optimalen Verteilung der Partikel im Luftstrom bei. Das Desinfektionsmittel wird dem Luftstrom in Strömungsrichtung vor der Austrittsöffnung zugeführt. Dadurch lässt sich das Desinfektionsmittel sehr fein verteilen, so dass es die Hände des Anwenders optimal flächig benetzt. Zudem kann der Verbrauch an Desinfektionsmittel gering gehalten werden.

Der Strömungsraum endet vorteilhaft mit Abstand vor der Austrittsöffnung, so dass die Luft vor dem Austritt aus dem Händetrockner beruhigt wird, was zu einer gleichmäßigen Luftströmung führt.

Bei einer bevorzugten Ausführungsform weist der Händetrockner mehrere Austrittsöffnungen auf, deren Achsen bzw. Ausströmrichtungen winklig zueinander liegen. Dadurch wird der Luftstrom in einzelne Teilströme aufgeteilt, so dass die Hände des Anwenders zuverlässig getrocknet werden. Je nach Winkellage der Austrittsöffnungen können die aus ihnen austretenden Luftströme so gelenkt werden, dass sie in einem kleineren oder größeren Bereich zusammentreffen, in dem die Trocknung der Hände erfolgt.

Vorteilhaft liegen die Achsen der Austrittsöffnungen unter spitzen Winkeln zueinander. Dadurch kann bei kleinen Abmessungen des Händetrockners ein großer Trocknungsbereich erzeugt werden.

Damit eine einfache und rasche Trocknung der Hände möglich ist, treffen die aus den Austrittsöffnungen austretenden Luftströme in einem Trocknungsbereich zusammen, der mit Abstand zu den Austrittsöffnungen liegt.

Der Behälter für das Desinfektionsmittel kann im Bereich neben dem Gehäuse oder innerhalb des Gehäuses vorgesehen sein. Befindet sich der Behälter im Bereich neben dem Gehäuse, kann er einfach ausgetauscht bzw. nachgefüllt werden. Befindet sich der Behälter innerhalb des Gehäuses, dann ist er vor Beschädigung und/oder Verschmutzung geschützt.

Der Händetrockner ist bei einer vorteilhaften Ausführungsform mit zwei unabhängigen, berührungslosen Sensoren versehen, mit denen der Trocknungsprozess und das Sprühen des Desinfektionsmittels aktiviert werden können.

Das Desinfektionsmittel wird bei einer vorteilhaften Ausbildung automatisch nach dem Trocknen der Hände aufgesprüht.

Bei einer anderen vorteilhaften Ausführungsform ist der Händetrockner so ausgebildet, dass er das Desinfektionsmittel mit Beginn des Trocknungsvorganges auf die Hände sprüht.

Der Händetrockner kann bei einer weiteren bevorzugten Ausbildung auch so ausgebildet sein, dass der Benutzer wahlweise das Sprühen des Desinfektionsmittels aktivieren kann. Er hat dadurch die Möglichkeit, das Desinfektionsmittel vor oder nach dem Trocknungsprozess auf seine Hände zu sprühen oder auf die Desinfizierung der Hände überhaupt zu verzichten.

Ein gleichmäßiger Sprühauftrag und eine gleichmäßige Trocknung der Hände wird in vorteilhafter Weise bei einfacher konstruktiver Gestaltung des Händetrockners erreicht, wenn die Austrittsöffnungen und die Austrittsdüsen für das Desinfektionsmittel konzentrisch zueinander liegen.

Wenn der Händetrockner einen programmierbaren Mikroprozessor enthält, können verschiedenen Sequenzen für den Trocknungsprozess und des Desinfiziervorgang einfach vorgenommen werden. Diese Programmierung kann so gestaltet sein, dass sie vom Anwender je nach seinen Anforderungen selbst vorgenommen werden kann.

Der Anmeldungsgegenstand ergibt sich nicht nur aus dem Gegenstand der einzelnen Ansprüche, sondern auch durch alle in den Zeichnungen und der Beschreibung offenbarten Angaben und Merkmale. Sie werden, auch wenn sie nicht Gegenstand der Ansprüche sind, als erfindungswesentlich beansprucht, soweit sie einzeln oder in Kombination gegenüber dem Stand der Technik neu sind.

Weitere Merkmale der Erfindung ergeben sich aus den weiteren Ansprüchen, der Beschreibung und den Zeichnungen.

Die Erfindung wird anhand zweier in den Zeichnungen dargestellter Ausführungsformen näher erläutert. Es zeigen
- Fig. 1: in schematischer Darstellung und im Längsschnitt eine erste Ausführungsform eines erfindungsgemäßen Händetrockners,
- Fig. 2: eine Ansicht in Richtung des Pfeiles II in Fig. 1,
- Fig. 3: in einer Darstellung entsprechend Fig. 1 eine zweite Ausführungsform eines erfindungsgemäßen Händetrockners.

Der Händetrockner ist so ausgebildet, dass er nicht nur zum Trocknen, sondern auch zum Desinfizieren der Hände eingesetzt werden kann. Der Händetrockner ist beispielhaft an einer Montageplatte 1 befestigt, die an einer Wand o. dgl. befestigt, beispielsweise angeschraubt ist. Der Händetrockner hat ein Gehäuse 2, in dem ein Gebläsemotor 3 untergebracht ist, mit dem in bekannter Weise ein Luftstrom erzeugt wird, der unten aus dem Gehäuse 2 austritt.

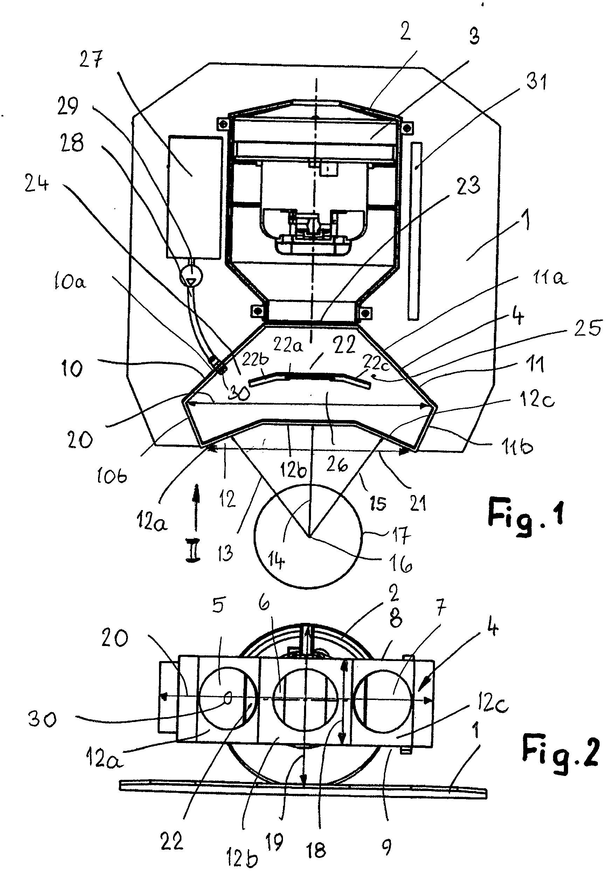

An das untere Gehäuseende ist eine Luftführung 4 angeschlossen, die rechteckigen Querschnitt aufweist (Fig. 2) und mit drei Austrittsöffnungen 5 bis 7 versehen ist. Sie haben jeweils kreisförmigen Querschnitt und sind vorteilhaft gleich groß.

Die Luftführung 4 erweitert sich vom Gehäuse 2 aus in Strömungsrichtung der Luft zunächst stetig. Die Luftführung 4 hat zueinander parallele Längsseitenwände 8, 9, die durch schmale Seitenwände 10, 11 miteinander verbunden sind. Diese Seitenwände bestehen aus zwei stumpfwinklig zueinander liegenden Wandabschnitten 10a, 10b; 11a, 11b. Die Wandabschnitte 10a, 11a schließen an das Austrittsende des Gehäuses 2 an und divergieren in Strömungsrichtung der Luft. Diese Wandabschnitte 10a, 11a sind länger als die Wandabschnitte 10b, 11b, die in Strömungsrichtung der Luft konvergierend zueinander verlaufen. Die Wandabschnitte 10b, 11b sind durch eine Abschlusswand 12 miteinander verbunden, die auch die Längsseitenwände 8, 9 der Luftführung 4 miteinander verbindet. Die Abschlusswand 12 besteht aus drei stumpfwinklig zueinander liegenden Wandabschnitten 12a, 12b, 12c. Die Wandabschnitte 12a, 12c schließen etwa rechtwinklig an die Wandabschnitte 10b, 11b an und liegen in Strömungsrichtung der Luft divergierend zueinander. Der mittlere Wandabschnitt 12b liegt mittig und senkrecht zur Längsachse des Gehäuses 2.

In jedem Wandabschnitt 12a bis 12c ist jeweils eine der Austrittsöffnungen 5 bis 7 vorgesehen. Da die Wandabschnitte 12a bis 12c winklig zueinander liegen, tritt die vom Gebläsemotor 3 erzeugte Luft in drei winklig zueinander liegenden Luftströmungen 13 bis 15 aus der Luftführung 4 aus. In Fig. 1 sind diese verschiedenen Luftströme der Einfachheit halber jeweils durch eine Gerade gekennzeichnet. Die Wandabschnitte 12a bis 12c sowie die darin vorgesehenen Austrittsöffnungen 5 bis 7 sind so ausgerichtet, dass die Luftströme 13 bis 15 in einer Trocknungsposition 16 zusammentreffen. In diesem Bereich befinden sich beim Einsatz des Händetrockners die zu trocknenden Hände. Die Wandabschnitte 12a bis 12c sind so ausgerichtet, dass die Trocknungsposition 16 nur einen solchen Abstand von den Luftaustrittsöffnungen 5 bis 7 hat, dass ein ausreichend starker Luftstrom auf die zu trocknenden Hände trifft, so dass eine wirksame Händetrocknung sichergestellt ist. Die zu trocknenden Hände befinden sich in dem durch einen Kreis 17 in Fig. 1 schematisch dargestellten Trocknungsbereich, der so groß ist, dass die Hände vollständig oder in erheblichem Maße erfasst werden. Die Luftströme 13 bis 15 bilden im Trocknungsbereich 17 vorteilhaft ein Wirbelfeld, das zu einer raschen Trocknung beiträgt.

Die Luftführung 4 hat eine Breite 18, die kleiner ist als die Breite 19 des Gehäuses 2. Es hat im Ausführungsbeispiel vorteilhaft kreisförmigen Querschnitt, so dass die Breite 19 dem Durchmesser des Gehäuses 2 entspricht. Das Gehäuse 2 kann aber auch jeden anderen geeigneten Querschnitt aufweisen.

Die maximale Länge 20 der Luftführung 4 ist größer als die Gehäusebreite 19. Auch im Austrittsbereich ist die Länge 21 der Luftführung 4 größer als die Gehäusebreite 19.

Es wird darauf hingewiesen, dass diese Abmessungsverhältnisse nicht zwingend sind, sondern dass die Luftführung 4 auch andere Abmessungen aufweisen kann, je nach Einsatzgebiet des Händetrockners. Auch die beschriebene Formgestaltung der Luftführung 4 ist zwar vorteilhaft, jedoch kann die Luftführung insgesamt auch eine andere Gestaltung haben. Es ist auch möglich, dass die Luftführung am Austrittsende nur eine einzige Austrittsöffnung, nur zwei oder auch mehr als drei Austrittsöffnungen aufweist. Die Luftführung und die entsprechende Zahl von Austrittsöffnungen ist stets so vorzusehen, dass die im Trocknungsbereich 17 befindlichen Hände innerhalb kurzer Zeit rasch und zuverlässig getrocknet wird.

Innerhalb der Luftführung 4 befindet sich eine Verteilerplatte 22, durch welche die aus dem Austrittsende 23 des Gehäuses 2 austretende und in die Luftführung 4 gelangende Luft so verteilt wird, dass aus den Austrittsöffnungen 5 bis 7 jeweils etwa die gleiche Luftmenge austritt. Die Verteilerplatte 22 erstreckt sich zwischen den beiden Längsseitenwänden 8, 9 der Luftführung 4 und ist an ihnen in geeigneter Weise befestigt. Die Verteilerplatte 22 liegt mittig in der Luftführung und hat einen mittleren Abschnitt 22a, der parallel zum mittleren Wandabschnitt 12b verläuft und im Bereich oberhalb der mittleren Austrittsöffnung 6 liegt. An den mittleren Plattenabschnitt 22a schließen stumpfwinklig zwei Abschnitte 22b und 22c an, die in Richtung auf die Abschlusswand 12 der Luftführung 4 divergierend verlaufen. Die Abschnitte 22b, 22c liegen mit Abstand zu den Wandabschnitten 10a, 11a und liegen spitzwinklig zu ihnen. Dadurch wird zwischen den Abschnitten 22b, 22c und den mit Abstand gegenüberliegenden Wandabschnitten 10a, 10b jeweils ein Strömungsraum 24, 25 gebildet, der sich in Strömungsrichtung der Luft verengt. Dadurch wird die Luft in den Strömungsräumen 24, 25 beschleunigt. Vorteilhaft ist die Ausströmgeschwindigkeit größer als etwa 35 m/s.

Die Verteilerplatte 22 sorgt dafür, dass der aus dem Austrittsende 23 austretende Luftstrom gleichmäßig so aufgeteilt wird, dass durch die Austrittsöffnungen 5 bis 7 etwa gleiche Luftmengen austreten. Die durch die Strömungsräume 24, 25 strömende Luft gelangt in einen einheitlichen Strömungsraum 26, der sich im Bereich oberhalb der Austrittsöffnungen 5 bis 7 befindet und der in Strömungsrichtung nach unten durch die Abschlusswand 12 und entgegengesetzt hierzu teilweise durch die Verteilerplatte 22 begrenzt wird.

Der Luftstrom wird in bekannter Weise erwärmt, wofür der Händetrockner mit einem Warmluftstromregler ausgestattet ist.

Im dargestellten Ausführungsbeispiel sind die Austrittsöffnungen 5 bis 7 in einer Linie nebeneinander angeordnet (Fig. 2). Es ist aber auch möglich, die Austrittsöffnungen so anzuordnen, dass ihre Achsen beispielsweise auf einem Kreis liegen. Dabei liegen die Achsen so winklig zueinander, dass die Luftströme im Trocknungsbereich 17 zusammentreffen.

Die Zahl der Austrittsöffnungen richtet sich auch nach deren Durchmesser. Je kleiner der Öffnungsdurchmesser der Austrittsöffnungen 5 bis 7 ist, desto mehr Austrittsöffnungen werden vorgesehen, damit die erforderliche Luftmenge in den Trocknungsbereich 17 gelangt.

Die Austrittsöffnungen 5 bis 7 sind düsenartig ausgebildet und können außer den beschriebenen Formen auch als Raster- oder Lochblenden oder als Schlitze ausgebildet sein. Die Austrittsöffnungen können auch durch eine Vielzahl kleiner Bohrungen und/oder kleiner Schlitze in der Wand 12 gebildet sein.

Die Anordnung der Austrittsöffnungen 5 bis 7 wird vorteilhaft so gewählt, dass die Luftströme leicht schräg vom Benutzer weg aus den Austrittsöffnungen austreten.

Dem Luftstrom wird ein Desinfektionsmittel zugeführt, das sich in einem Tank 27 befindet, der beispielsweise als Behälter ausgebildet ist, der leicht nachgefüllt oder auch ausgetauscht werden kann, wenn das Desinfektionsmittel aufgebraucht ist. Um ein einfaches Austauschen bzw. Nachfüllen zu ermöglichen, ist der Tank 27 vorteilhaft neben dem Gehäuse 2 an der Montageplatte 1 gehalten. Grundsätzlich kann der Tank 27 auch innerhalb des Gehäuses 2 angeordnet werden. Unter Desinfektionsmittel wird im Folgenden das Lösungsmittel und das darin befindliche Desinfektionsmedium verstanden.

An den Tank 27 ist wenigstens eine Leitung 28 über ein Ventil 29 angeschlossen. Am freien Ende der Leitung 28 befindet sich eine Austrittsdüse 30, die am Wandabschnitt 10a der Luftführung 4 befestigt ist und aus der das Desinfektionsmittel austritt. Die Austrittsdüse 30 ist so vorgesehen, dass das austretende Desinfektionsmittel in den durch den Strömungsraum 24 strömenden Teilluftstrom gelang. Da die Strömungsgeschwindigkeit infolge der Querschnittsverringerung des Strömungsraumes 24 hoch ist, wird das Desinfektionsmittel beim Austritt aus der Austrittsdrüse 30 mitgerissen und fein im Luftstrom verteilt. Dadurch tritt das Desinfektionsmittel fein verteilt durch die Austrittsöffnung 5 bis 7 aus und gelangt gleichmäßig auf die zu desinfizierenden Hände, die sich im Trocknungsbereich 17 befinden.

In Fig. 1 ist schematisch die Elektronik 31 dargestellt, mit der der Händetrockner betätigt wird.

Beim dargestellten Ausführungsbeispiel ist nur eine Austrittsdüse 30 für das Desinfektionsmittel vorgesehen. Es ist auch möglich, am gegenüberliegenden Wandabschnitt 11a in gleicher Weise eine weitere Austrittsdüse vorzusehen, so dass das Desinfektionsmittel an zwei Stellen in den Luftstrom eingebracht werden kann. Diese weitere Austrittsdüse kann über eine weitere Leitung mit dem Tank 27 verbunden werden. Es ist grundsätzlich auch möglich, für die weitere Austrittsdüse einen weiteren Tank für das Desinfektionsmittel vorzusehen.

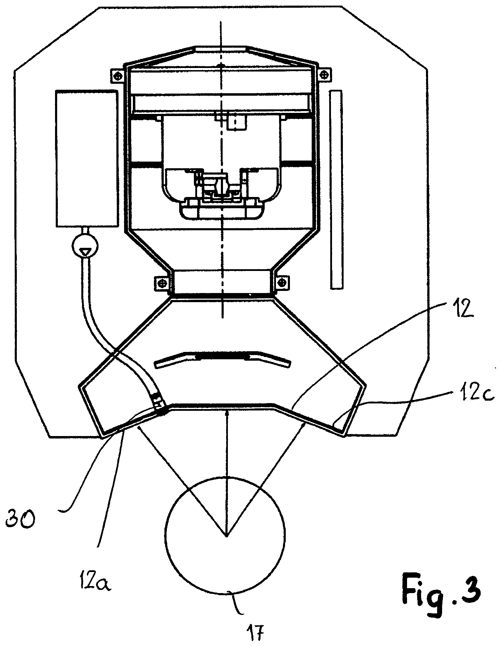

Bei der Ausführungsform gemäß Fig. 3, die nicht in den Rahmen der vorliegenden Erfindung fällt, ist die Austrittsdüse 30 am Wandabschnitt 12a befestigt und so angeordnet, dass das Desinfektionsmittel unmittelbar auf die im Trocknungsbereich 17 befindlichen Hände gesprüht wird. Die Austrittsdüse 30 ist in diesem Falle im Bereich neben der entsprechenden Austrittsöffnung in der Abschlusswand 12 befestigt. Wie beim vorigen Ausführungsbeispiel beschrieben, kann eine weitere Austrittsdüse auch am Wandabschnitt 12c vorgesehen sein, so dass das Desinfektionsmittel von zwei Stellen aus in den Trocknungsbereich 17 gesprüht wird.

Mit dem beschriebenen Händetrockner ist es nicht nur möglich, die Hände nach einem Waschvorgang zu trocknen, sondern auch zu desinfizieren und anschließend zu trocknen. Die Elektronik 31 ist so gestaltet, dass unterschiedliche Verfahrensweisen beim Einsatz des Händetrockners möglich sind. Der Händetrockner wird z. B. in Manipulations- und Operationsräumen in Krankenhäusern, im Bereich der Tiermedizin und dort eingesetzt, wo der Arzt sterile Handschuhe verwendet.

Der Händetrockner ist vorteilhaft so ausgebildet, dass er selbsttätig dann eingeschaltet wird, wenn sich die Hände im Trocknungsbereich 17 befinden. Eine solche selbsttätige Einschaltung ist bei Händetrocknern bekannt und wird darum auch nicht näher beschrieben. Sobald die Hände aus dem Trocknungsbereich 17 herausgezogen werden, wird der Händetrockner automatisch abgeschaltet.

Die Elektronik 31 kann bei einer ersten Ausführungsform so ausgebildet sein, dass zunächst mit dem Gebläsemotor 3 die Trocknungsluft erzeugt wird, die aus den Austrittsöffnungen 5 bis 7 austritt und auf die im Trocknungsbereich 17 befindlichen Hände trifft, wodurch diese getrocknet werden. Nach einer vorgegebenen Zeit wird die Trocknungstemperatur verringert und auch die Strömungsgeschwindigkeit der Trocknungsluft herabgesetzt und dann automatisch die Zuführung des Desinfektionsmittels eingeschaltet. Dem Tank 27 ist eine (nicht dargestellte) Dosiereinrichtung zugeordnet, mit der das Desinfektionsmittel aus dem Tank 27 unter Druck in die Leitung 28 gefördert werden kann. Hierzu wird das Ventil 29 gesteuert eine entsprechende Zeit geöffnet. Das Desinfektionsmittel wird über die Austrittsdüse 30 in den Luftstrom gesprüht, der das Desinfektionsmittel aufgrund seiner entsprechend hohen Geschwindigkeit ausreichend fein verteilt. Das Gemisch aus Luft und Desinfektionsmittel gelangt über die Austrittsöffnungen 5 bis 7 auf die im Trocknungsbereich 17 befindlichen Hände. Nach einer vorgegebenen Zeit wird die Zufuhr des Desinfektionsmittels automatisch abgeschaltet und nur noch die Trocknungsluft so lange zugeführt, bis das Lösungsmittel verdunstet ist. Vorteilhaft wird die Trocknungsluft so lange zugeführt, bis die Hände aus dem Trocknungsbereich 17 zurückgezogen werden und der Gebläsemotor 3 automatisch abgeschaltet wird. Während der Trocknungsphase nach dem Desinfizieren kann es von Vorteil sein, wenn die Strömungsgeschwindigkeit der Trocknungsluft wieder erhöht wird.

Beim Ausführungsbeispiel nach Fig. 3, das nicht in den Rahmen der vorliegenden Erfindung fällt, wird das Desinfektionsmittel nicht in den Luftstrom innerhalb der Luftführung 4 eingegeben, sondern unmittelbar in Richtung auf den Trocknungsbereich 17 gesprüht. Das Desinfektionsmittel wird von den aus den Austrittsöffnungen 5 bis 7 austretenden Luftströmen mitgerissen. Im Übrigen erfolgt der Trocknungsvorgang in gleicher Weise, wie zuvor beschrieben. Bei dieser Ausführungsform ist es auch möglich, zunächst nur das Desinfektionsmittel auf die Hände zu sprühen und erst dann die Trocknungsluft zu erzeugen. Der Händetrockner ist in diesem Fall so ausgebildet, dass der Anwender zunächst die Ausbringung des Desinfektionsmittels auslöst. Dann wird der Gebläsemotor 3 zeitgesteuert eingeschaltet oder vom Anwender eingeschaltet.

Der Händetrockner kann auch so ausgebildet sein, dass mit ihm nur ein Desinfektionsvorgang durchgeführt wird. Sobald die Hände in den Trocknungsbereich 17 gelangen, wird die Zufuhr des Desinfektionsmittels aus dem Tank 27 automatisch eingeschaltet, so dass das Desinfektionsmittel entweder in den Luftstrom innerhalb der Luftführung eingesprüht (Fig. 1 und 2) oder direkt in die im Trocknungsbereich 17 befindlichen Hände gesprüht wird. Anschließend erfolgt die Händetrocknung, indem die Zuführung des Desinfektionsmittels abgeschaltet wird und nur noch die Trocknungsluft über die Austrittsöffnungen 5 bis 7 in den Trocknungsbereich 17 gefördert wird. Hierbei wird das Lösungsmittel des Desinfektionsmittels auf den Händen verdunstet und die Hände somit getrocknet. Sobald die desinfizierten Hände aus dem Trocknungsbereich 17 zurückgezogen werden, schaltet der Gebläsemotor 3 ab.

Die Zuführung des Desinfektionsmittels über den Händetrockner hat den Vorteil, dass das Desinfektionsmittel fein verteilt auf die Hände gelangt. Die Trocknungsluft sorgt für eine kurze Trocknung der Hände. Da das Desinfektionsmittel über die Austrittsdüse 30 gesprüht wird, wird auch Desinfektionsmittel eingespart. Aufgrund des Sprühvorganges werden die zu desinfizierenden Hände optimal gleichmäßig besprüht, so dass die Desinfizierung zuverlässig erfolgt.

Der Händetrockner kann mit einem Zeitschalter, einem Luftstromregler oder einem Warmluftstromregler ausgestattet sein.

Der Trocknungsprozess und das Sprühen des Desinfektionsmittels kann vorteilhaft durch zwei unabhängige, berührungslose Sensoren aktiviert werden. Sobald die Hände in den Trocknungsbereich 17 gelangen, wird über diese berührungslosen Sensoren der Gebläsemotor 3 eingeschaltet und das Desinfektionsmittel gesprüht. Eine solche Steuerung des Händetrockners ist bei den beiden beschriebenen Ausführungsformen gemäß den Fig. 1, 2 und 3 möglich.

Es ist weiter möglich, den Händetrockner so auszubilden, dass das Sprühen des Desinfektionsmittels auf Wunsch des Anwenders aktiviert werden kann. Dann kann der Anwender selbst bestimmen, ob das Desinfektionsmittel vor oder nach dem Trocknungsprozess oder überhaupt nicht auf die Hand gesprüht wird.

Vorteilhaft können die Austrittsdüsen 30 konzentrisch zueinander liegen, so dass die Hände gleichmäßig beim Trocknungsvorgang mit dem Luftstrom beaufschlagt und das Desinfektionsmittel gleichmäßig aufgesprüht wird.

Der Handtrockner kann mit einem Mikroprozessor ausgerüstet sein, mit dem unterschiedliche Sequenzen für den Trocknungsprozess und den Desinfizierprozess programmiert werden können.

## Patentansprüche

1. Händetrockner mit einem Gehäuse (2), in dem wenigstens ein Gebläsemotor (3) untergebracht ist, mit dem Luft durch wenigstens eine Austrittsöffnung (5 bis 7) nach außen geblasen wird, wobei dem Luftstrom ein Desinfektionsmittel über wenigstens eine an einer Düse vorgesehene Austragöffnung (5 bis 7) zuführbar ist, wobei der Händetrockner eine Luftführung (4) umfasst, die wenigstens einen Strömungsraum (24, 25) aufweist, wobei die wenigstens eine Austragöffnung (5 bis 7) in einer Wand (10, 12) der Luftführung (4) vorgesehen ist, und wobei in der Luftführung (4) eine Verteilerplatte (22) angeordnet ist, die im Strömungsweg der Luft liegt und zusammen mit der Wand (10, 12) den wenigstens einen Strömungsraum (24, 25) begrenzt, dessen Querschnitt in Strömungsrichtung der Luft abnimmt, **dadurch gekennzeichnet, dass** die Düse (30) für den Austrag des Desinfektionsmittels im Bereich des Strömungsraumes (24, 25) angeordnet ist, dass die Austragöffnung in einem Bereich der Luftführung (4) vorgesehen ist, in dem die Luft beschleunigt wird und dass das Desinfektionsmittel dem Luftstrom in Strömungsrichtung vor der Austrittsöffnung (5 bis 7) zugeführt wird.

2. Händetrockner nach Anspruch 1,
**dadurch gekennzeichnet, dass** der Strömungsraum (24, 25) mit Abstand von der Austrittsöffnung (5 bis 7) endet.

3. Händetrockner nach Anspruch 1 oder 2,
**dadurch gekennzeichnet, dass** der Händetrockner mehrere Austrittsöffnungen (5 bis 7) aufweist, deren Achsen bzw. Ausströmrichtungen winklig, vorzugsweise unter spitzen Winkeln zueinander liegen.

4. Händetrockner nach Anspruch 4,
**dadurch gekennzeichnet, dass** die aus den Austrittsöffnungen (5 bis 7) austretenden Luftströme in einem Trocknungsbereich (17) zusammentreffen, der mit Abstand zu den Austrittsöffnungen (5 bis 7) liegt.

5. Händetrockner nach Anspruch 1 bis 4,
**dadurch gekennzeichnet, dass** ein Behälter (27) für das Desinfektionsmittel im Bereich neben dem Gehäuse (2) vorgesehen ist.

6. Händetrockner nach einem der Ansprüche 1 bis 4,
**dadurch gekennzeichnet, dass** ein Behälter (27) für das Desinfektionsmittel im Gehäuse (2) vorgesehen ist.

7. Händetrockner nach einem der Ansprüche 1 bis 6,
**dadurch gekennzeichnet, dass** zum Aktivieren des Trocknungsprozesses und des Sprühens des Desinfektionsmittels zwei unabhängige, berührungslose Sensoren vorgesehen sind.

8. Händetrockner nach einem der Ansprüche 1 bis 7,
**dadurch gekennzeichnet, dass** das Desinfektionsmittel automatisch nach dem Trocknen der Hände aufgesprüht wird.

9. Händetrockner nach einem der Ansprüche 1 bis 8,
**dadurch gekennzeichnet, dass** das Desinfektionsmittel mit Beginn des Trocknungsvorganges auf die Hände aufgesprüht wird.

10. Händetrockner nach einem der Ansprüche1 bis 9,
**dadurch gekennzeichnet, dass** das Sprühen des Desinfektionsmittels vom Benutzer wahlweise aktivierbar ist.

11. Händetrockner nach einem der Ansprüche 1 bis 10,
**dadurch gekennzeichnet, dass** ein programmierbarer Mikroprozessor
zur Programmierung unterschiedlicher Sequenzen von Trocknen und Desinfizieren vorgesehen ist.

## Claims

1. A hand dryer with a housing (2), which incorporates at least one blower motor (3), with which air is blown out through at least one outlet opening (5 to 7), wherein a disinfectant can be supplied to the air stream via at least one discharge opening (5 to 7) provided on a nozzle, wherein the hand dryer comprises an air guide (4) having at least one flow space (24, 25), wherein the at least one discharge opening (5 to 7) is provided in a wall (10, 12) of the air guide (4), and wherein the air guide (4) incorporates a distributor plate (22) that lies in the flow path of the air, and together with the wall (10, 12) delimits the at least one flow space (24, 25) whose cross section tapers in the direction of air stream, **characterized in that** the nozzle (30) for discharging the disinfectant is arranged in the area of the flow space (24, 25), that the discharge opening is provided in an area of the air guide (4) in which the air is accelerated, and that the disinfectant is supplied to the air stream in the direction of flow before the discharge opening (5 to 7).

2. The hand dryer according to claim 1,
**characterized in that** the flow space (24, 25) ends spaced a distance apart from the discharge opening (5 to 7).

3. The hand dryer according to claim 1 or 2,
**characterized in that** the hand dryer has several discharge openings (5 to 7), whose axes or outflow directions are positioned at an angle, preferably at acute angles, to each other.

4. The hand dryer according to claim 4,
**characterized in that** the air streams exiting the discharge openings (5 to 7) converge in a drying area (17) spaced a distance apart from the discharge openings (5 to 7).

5. The hand dryer according to claim 1 to 4,
**characterized in that** a container (27) for the disinfectant is provided in the area adjacent to the housing (2).

6. The hand dryer according to one of claims 1 to 4,
**characterized in that** a container (27) for the disinfectant is provided in the housing (2).

7. The hand dryer according to one of claims 1 to 6,
**characterized in that** two independent, contactless sensors are provided for activating the drying process and spraying the disinfectant.

8. The hand dryer according to one of claims 1 to 7,
**characterized in that** the disinfectant is automatically sprayed on after drying the hands.

9. The hand dryer according to one of claims 1 to 8,
**characterized in that** the disinfectant is sprayed onto the hands at the beginning of the drying process.

10. The hand dryer according to one of claims 1 to 9,
**characterized in that** the user can optionally activate the spraying of disinfectant.

11. The hand dryer according to one of claims 1 to 10,
**characterized in that** a programmable microprocessor is provided for programming various drying and disinfecting sequences.

## Revendications

1. Sèche-mains avec un boîtier (2) dans lequel est logé au moins un moteur de soufflante (3) avec lequel de l'air est soufflé vers l'extérieur par au moins une ouverture de sortie (5 à 7), un désinfectant prouvant être dirigé vers le flux d'air par le biais d'au moins une ouverture d'extraction (5 à 7) prévue sur une buse, le sèche-mains comprenant un guidage d'air (4), qui comporte au moins un compartiment d'écoulement (24,25), une ouverture d'extraction (5 à 7) étant prévue dans une paroi (10,12) du guidage d'air (4) et une plaque de répartition (22) étant disposée dans le guidage d'air (4) qui se situe dans la trajectoire d'écoulement de l'air et délimite ensemble avec la paroi (10,12) au moins un compartiment d'écoulement (24,25), dont la section diminue dans la direction de l'écoulement de l'air, **caractérisé en ce que** la buse (30) pour l'extraction du désinfectant est disposée dans la zone du compartiment d'écoulement (24,25), **en ce que** l'ouverture d'extraction est prévue dans une zone du guidage d'air (4), l'air étant accéléré et **en ce que** le désinfectant est dirigé vers le flux d'air dans la direction d'écoulement devant l'ouverture de sortie (5 à 7).

2. Sèche-mains selon la revendication 1, **caractérisé en ce que** le compartiment d'écoulement (24,25) se termine à distance de l'ouverture de sortie (5 à 7).

3. Sèche-mains selon la revendication 1 ou 2, **caractérisé en ce que** le sèche-mains comporte plusieurs ouvertures de sortie (5 à 7) dont les axes ou direction d'écoulement se situent en angle l'un par rapport à l'autre, de préférence à angle aigu.

4. Sèche-mains selon la revendication 4, **caractérisé en ce que** les flux d'air sortant des ouvertures de sortie (5 à 7) se rencontrent dans une zone de séchage (17), qui se situe à distance des ouvertures de sortie (5 à 7).

5. Sèche-mains selon la revendication 1 à 4, **caractérisé en ce qu'**un réservoir (27) pour le désinfectant est prévu dans la zone se situant près du boîtier (2).

6. Sèche-mains selon la revendication 1 à 4, **caractérisé en ce qu'**un réservoir (27) pour le désinfectant est prévu dans le boîtier (2).

7. Sèche-mains selon l'une quelconque des revendications 1 à 6, **caractérisé en ce que** pour activer le processus de séchage et de pulvérisation du désinfectant deux capteurs indépendants, sans contact sont prévus.

8. Sèche-mains selon l'une quelconque des revendications 1 à 7, **caractérisé en ce que** le désinfectant est diffusé automatiquement après le séchage des mains.

9. Sèche-mains selon l'une quelconque des revendications 1 à 8, **caractérisé en ce que** le désinfectant est diffusé sur les mains au début de l'opération de séchage.

10. Sèche-mains selon l'une quelconque des revendications 1 à 9, **caractérisé en ce que** la pulvérisation du désinfectant peut être activée facultativement par l'utilisateur.

11. Sèche-mains selon l'une quelconque des revendications 1 à 10, **caractérisé en ce qu'**un microprocesseur programmable est prévu pour la programmation de séquences différentes de séchage et de désinfection.
